# EUROPÄISCHE PATENTSCHRIFT

(11) **EP 0 743 836 B1**
(45) Veröffentlichungstag und Bekanntmachung des Hinweises auf die Patenterteilung: **16.12.1998**
(21) Anmeldenummer: 95906870.1
(22) Anmeldetag: 02.02.1995
(51) Int. Cl.: A61B 17/08

(54) **EINSTÜCKIGE WUNDKLAMMER**
ONE-PIECE SURGICAL CLIP
AGRAFE EN UNE PIECE A USAGE MEDICAL

(30) Priorität: 11.02.1994 CH 415/94
(43) Veröffentlichungstag der Anmeldung: 27.11.1996
(73) Patentinhaber: WALDER-UTZ, Alice, Dr., CH-8006 Zürich (CH)
(72) Erfinder: WALDER-UTZ, Alice, CH-8006 Zürich (CH); DUBACH, Werner, Fritz, CH-8124 Maur (CH)
(74) Vertreter: Patentanwaltsbüro Feldmann AG
(86) Internationale Anmeldenummer: CH9500023
(87) Internationale Veröffentlichungsnummer: WO9521575

(56) Entgegenhaltungen:
- EP-A- 0 469 524
- WO-A-88/01487
- DE-C- 3 335 986
- FR-A- 471 440
- US-A- 2 910 067
- US-A- 5 047 047

## Beschreibung

Nach jeder chirurgischen Operation kommt der Wundheilung grosse Bedeutung zu. Man ist daher bestrebt, optimale Voraussetzungen für eine möglichst risikolose, schmerzfreie und schnelle Heilung zu schaffen. Es ist bekannt, dass Wunden, deren Schnittränder durch Klammern oder Fäden zusammengehalten werden, schneller und kosmetisch schöner heilen als Wunden, die man der Selbstheilung überlässt (Williams und Harrison 1977). Bei der Verwendung von Wundklammern ist die Infektionsrate niedriger, Granulome treten dabei nicht auf und die durchschnittliche Spitalaufenthaltsdauer des Patienten ist kürzer als bei der Verwendung von Fäden (Beresford et al. 1984). Bei Hautverbrennungen werden Klammern vorgezogen, weil sie viel schneller angebracht sind als eine Fadennaht. Das Hauttransplantat kann schneller plaziert werden und die kritische Anästhesiezeit kann daher verringert werden (Kahn et al. 1984, Hallock et al. 1984).

Durch die im Vergleich zur Fadennaht reduzierte Traumatisation des Gewebes zeigt die Klammerung eine schönere Wundheilung und eine geringere Narbenbildung. Nach Nockemann (1965/1968) hinterlassen Nähte an den Einstichstellen sowie an den Stellen, an denen der Faden eine zeitlang das Gewebe einschnürt oder drückt, eine Narbe. Entsprechend versucht man, die Einstichstellen nach Zahl und Grösse möglichst klein zu halten, um das bekannte Strickleiter-Syndrom möglichst zu vermeiden.

Nicht nur ist die Applikation und Entfernung von Wundklammern einfach und schnell, sondern auch die Gefahr der Keimansiedlung auf den Wundklammern ist gering. Im Gegensatz dazu erhöht die Verwendung von Nähmaterial in der Wunde das Risiko der Virulenz der Staphylokokken um einige 10000 mal. Die Abszessrate ist bei der Klammertechnik etwa dreimal niedriger. Nach Stephens (1990) zeigen mit Klammern verschlossene Wunden am siebenten postoperativen Tag bessere mechanische Eigenschaften als mit einer Fadennaht verschlossene. Das heisst, der Elastizitätsmodul ist grösser, die Zugfestigkeit höher und die Fähigkeit zur Wundabsorption ohne Ruptur besser. Lowdon et al. (1992) fand auch, dass die postoperativen Komplikationen in Form von Anastomosenschwäche, Wundinfektion und Blutungen bei Verwendung von Klammern um rund 25% seltener sind.

Herkömmliche Klammern für die Applikation an Körperteilen durchstechen die beiden zusammenzuhaltenden Hautseiten der Wunde an mehreren Stellen. Zum einen ist dieses Durchstechen für den Patienten schmerzhaft und zum anderen aus medizinischen Gründen ungünstig. Neben der kosmetischen Beeinträchtigung durch die Stiche erhöhen diese vor allem die Infektionsgefahr, da Fremdmaterial in die Wunde eindringt. Zudem erfordert die Entfernung der Klammern bei vielen Klammersystemen ein spezielles Gerät.

Die meisten Nachteile, die in Zusammenhang mit der Verwendung von Fadenmaterial auftreten, haben sich auch bei den neueren Systemen mit Heftklammern eingestellt. Trotzdem hat sich der Wundverschluss mittels Heftklammernsystemen stark verbreitet. Im Gegensatz zur Anlegung einer Nähnaht verlangt das Heftklammerverfahren geringere Routine und ist insbesondere dank den Heftklammersetzgeräten, in denen die Heftklammern magaziniert sind, äusserst schnell. Hingegen erfordert die Entfernung der Heftklammern ein spezielles Gerät.

Aus der Patentliteratur sind verschiedene Wundklammern mit relativ einfachem Aufbau bekannt. So zeigt die US-A-3'601'127 eine Wundklammer, die aus zwei halbrunden Klemmbacken besteht, die über einen federelastischen Mittelsteg miteinander verbunden sind. Die Andruckkanten der Klemmbacken sind gezahnt und perforieren die Wundränder.
Demgegenüber zeigt die EP-A-224'500 eine Wundklammer, die aus zwei zueinander beweglichen Klemmbacken mit je einem Griff besteht, wobei die Andruckkanten gewellt sind. Eine Perforation der Wundränder tritt somit nicht auf.

Aus der FR-A-419'096 sind einstückige, federelastische, bogenförmige Wundklammern mit zwei zueinander gerichteten Andruckkanten bekannt, wobei zwei über einen Steg miteinander verbundene Klemmbacken die Andruckkanten bilden. Die Einfachheit dieser Wundklammern ist zwar bestechend, doch verlangen sie spezielle Zangen, um gesetzt zu werden. Zudem muss jede Klammer einzeln in die Zange eingelegt werden, bevor sie gesetzt werden kann.

Die WO-88/01487 zeigt Wundklammern gemäss Oberbegriff des Patentanspruches 1, die sich mittels den Andruckkanten auf einem die Wundklammer durch eine Oeffenung im Steg durchsetzenden Magazinierelement halten. Ein von der Oeffenung zur einer Andruckkante erstreckender Schlitz dient der Zuführung zu einem Spreizer.

Die vorliegende Erfindung stellt sich daher zur Aufgabe, eine einstückige, federelastische, bogenförmige Wundklammer mit zwei zueinander gerichteten Andruckkanten zu schaffen, die von zwei über einen Steg miteinander verbundenen Klemmbacken gebildet ist, so dass sie geeignet ist, um in einer Vielzahl in einem Wundklammernsetzgerät aufgenommen zu werden.

Diese Aufgabe wird in der generellsten Form dadurch gelöst, dass die Wundklammer Mittel zur stapelbaren Halterung aufweist.

In einer bevorzugten Ausführungsform besteht das Mittel zur stapelbaren Halterung aus einem länglichen, zentrischen Einschnitt, der den Steg und die beiden Klemmbacken senkecht zu den Andruckkanten durchsetzt. Derartig gestaltete wundklammern lassen sich dann auf ein speziell gestaltetes Magazinierelement wie auf einem Spiess stapeln.

Eine Alternative besteht darin, dass man als Mittel zur stapelbaren Halterung an mindestens einer der beiden seitlichen Stirnflächen der Wundklammer eine Ausnehmung einlässt, die sowohl den Steg als auch die beiden Klemmbacken über die Andruckkanten hinweg durchsetzt. In diesem Fall lassen sich die Wundklammern über einseitig oder beidseitig anliegende Magzinierelemente stapeln und gerichtet führen.

Damit sich die so magazinierten Wundklammern mittels einem Magazinierelement auch noch applizieren lassen, ist es vorteilhaft, wenn an den Innenflächen der Klemmbacken Mittel zur Halterung der Wundklammer in einer gespreizten Lage angebracht sind. Besonders einfach lässt sich dies realisieren, indem man diese Mittel als distanziert von den Andruckkanten verlaufende Rückhalterippen ausbildet.

Weitere vorteilhafte Ausführungsformen gehen aus den weiteren abhängigen Patentansprüchen hervor und sind in der nachfolgenden Beschreibung erläutert.

In der beiliegenden Zeichnung sind verschiedene Ausführungsformen des Erfindungsgegenstandes dargestellt, die in der nachfolgenden Beschreibung erklärt sind. Es zeigt:
- Figur 1 -: eine bevorzugte Ausführungsform der erfindungsgemässen Wundklammer in perspektivischer Darstellung;
- Figur 2 -: einen mittigen Querschnitt durch dieselbe Wundklammer senkrecht zu den Andruckkanten;
- Figur 3 -: einen mittigen Längsschnitt parallel zu den Andruckkanten und
- Figur 4 -: eine Aufsicht auf die Wundklammer nach den Figuren 1-3 in kleinerem Massstab.
- Figur 5 -: zeigt eine andere Ausführungsform der Wundklammer mit zwei seitlichen Ausnehmungen zur stapelbaren Halterung.
- Figur 6 -: zeigt eine Wundklammer entsprechend der Ausführung gemäss den Figuren 1-4, welche zusätzlich Spreizelemente aufweist.
- Figur 7 -: zeigt mehrere Wundklammern in gestapelter Anordnung aufgereiht auf einem Magazinierelement.

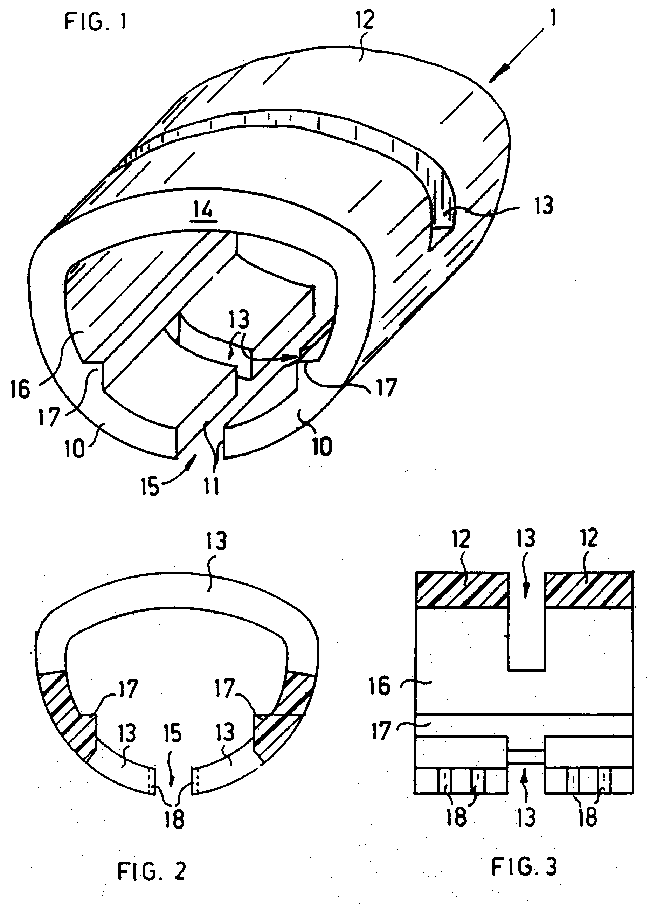

Die bevorzugte Ausführungsform der erfindungsgemässen Wundklammer, wie sie in den Figuren 1-4 dargestellt ist, ist aus Kunststoff gefertigt. Die Darstellung gemäss den Figuren 1-3 zeigt die Wundklammer in ca. 10 - 15-facher Vergrösserung. Prinzipiell liesse sich dieselbe Gestaltung auch aus einem Medizinalstahl fertigen. Bevorzugt wird jedoch die Wundklammer aus einem transparenten Kunststoff hergestellt, um während des Heilprozesses eine Sichtkontrolle der Naht zu gewährleisten. Die Wundklammer besteht im wesentlichen aus zwei Klemmbacken 10, die über den gemeinsamen Steg 12 miteinander einstückig verbunden sind. Die Endbereiche der Klemmbacken 10 bilden die Andruckkanten 11, die gemeinsam einen Klemmspalt 15 definieren. Die beiden Klemmbacken 10 und der Steg 12 bilden zusammen ein hohlzylindrisches Element mit etwa ovalem Querschnitt. Die beiden Andruckkanten 11 verlaufen parallel zur Längsachse dieses hohlzylindrischen Elementes. Senkrecht zur Längsachse des hohlzylindrischen Elementes ist ein zentrischer Einschnitt 13 angebracht, der den Steg 12 vollständig und die beiden Klemmbacken 10 teilweise durchsetzt. Im Bereich der Klemmbacken 10 erstreckt sich der zentrische Einschnitt über den Klemmspalt 15 hinweg und unterteilt somit die beiden Andruckkanten 11. Während somit die Querschnittsform der Wundklammer beidseits des zentrischen Einschnittes 13 etwa C-förmig ist, verbleibt im Bereich des zentrischen Einschnittes 13 lediglich beidseits eine massive Verbindungsbrücke der jeweiligen Klemmbacke 10 bestehen. Dies ist am deutlichsten in der Figur 2 zu erkennen. Da die Andruckkanten 11 durch den zentrischen Einschnitt 13 unterteilt sind, dürfen die Andruckkanten 11 auch geradlinig verlaufen. Um jedoch eine besonders gute Durchblutung auch im Klemmbereich zu gewährleisten, wird man vorteilhafterweise die Andruckkanten 11 mit wellenförmigen Vertiefungen 18 versehen, wie dies in den Figuren 2 und 3 dargestellt ist. Die Gestaltungsform der Andruckkanten 11 lässt sich jedoch beliebig variieren.

Wiederum parallel zur Längsachse der Wundklammer, parallel zu den Andruckkanten 11 und senkrecht zur Verlaufsrichtung des zentrischen Einschnittes 13 ist an den Innenflächen 16 der Klemmbacken 10 je eine Rückhalterippe 17 angeformt. Der zentrische Einschnitt 13 im Bereich der Klemmbacken 10 erstreckt sich lediglich bis zu den Rückhalterippen 17, durchsetzt diese aber nicht. Auf die Bedeutung und Funktion der Rückhalterippen 17 wird später eingegangen.

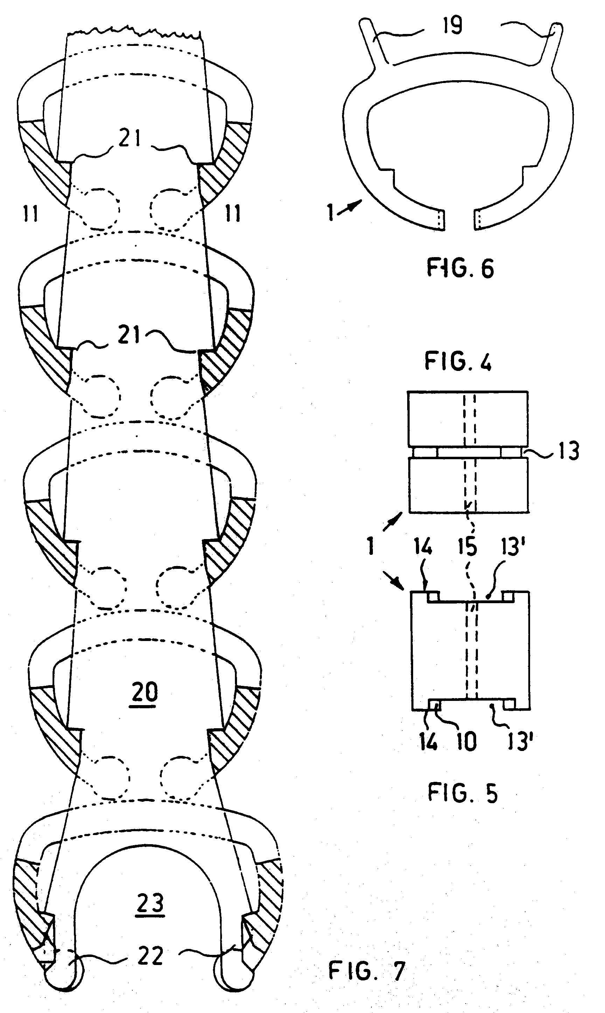

Während bei der bisher beschriebenen Ausführung das Mittel zur gestapelten Halterung der Wundklammer 1 aus einem zentrischen Einschnitt 13 bestanden hat, ist in der Figur 5 eine Variante dargestellt, bei der die Klammer nicht mittig durchsetzt ist, sondern eine entsprechende seitliche Ausnehmung 13' aufweist, die dieselbe Funktion übernehmen kann. In der Ausführung gemäss der Figur 5 sind die seitlichen Ausnehmungen 13' in beiden seitlichen Stirnflächen 14 der Wundklammer angebracht. Je nach der Gestaltung des Magazinierelementes kann jedoch auch eine einzige seitliche Ausnehmung 13' genügen. Zur positionierten Lagerung der Wundklammern 1 gemäss der Variante nach Figur 5 würde man diese in eine im Querschnitt rechtwinklige oder U-förmige Schiene einlegen und einseitig oder beidseitig ein Magazinierelement anordnen. Das Magazinierelement würde die Wundklammern positioniert gestapelt halten und mittels demselben liessen sich die Wundklammern schrittweise transportieren. Die seitlichen Ausnehmungen 13' erstrecken sich weitgehend über den Steg 12 hinweg und durchsetzen die darunter liegenden Klemmbacken 10 ebenfalls. Genau wie bei der vorher beschriebenen Variante, wo der zentrische Einschnitt 13 im Steg 12 länger ist als die Summe der beiden Einschnitte in den beiden Klemmbacken 10, ist auch die Länge der Ausnehmung 13' im Steg 12 länger als die Summe der beiden seitlichen Ausnehmungen aus den beiden darunter befindlichen Klemmbacken 10. Beide Varianten der Wundklammern gemäss den Figuren 4 und 5 lassen sich zusätzlich mit Spreizelementen 19 versehen, wie dies die Wundklammer 1 gemäss Figur 6 zeigt. Die Spreizelemente 19 bestehen aus zwei radial von der Oberfläche des Steges 12 abstehenden Wändchen. Diese sind nahe dem Ansatzbereich der Klemmbacken 10 am Steg 12 angeordnet. Die Wundklammern lassen sich an den Spreizelementen 19 fassen, wobei ein Druck gegeneinander ausgeübt werden kann, der zu einer gewissen Spreizung der Wundklammer führt. Hierdurch lassen sich zwar nicht genügend Kräfte auf die Wundklammer ausüben, um die Klemmbacken genügend weit zu spreizen, um die Wundklammer zu setzen, doch genügt die damit erzielbare Spreizung, um die Wundklammer ohne spezielle Hilfsmittel von der verheilten Naht zu entfernen. Die Anordnung der Spreizelemente 19 behindert deren Stapelfähigkeit nicht. Ist die Wundklammer mit einem zentralen Einschnitt 13 versehen, so erstreckt sich dieser Einschnitt auch durch die beiden Spreizelemente. Ist jedoch die Wundklammer gemäss der Variante nach Figur 5 gefertigt, so erstrecken sich die beiden Spreizelemente 19 hier quer über den Steg 12 von einer seitlichen Aussparung zu anderen. Die spezielle Ausgestaltung der erfindungsgemässen Wundklammer erlaubt es nun, nicht nur die Klammern zu stapeln, sondern insbesondere auch auf ein darauf angepasstes Magazinierelement 20 in einer fest vorgegebenen Lage zu positionieren. Dies ist in der Figur 7 anschaulich dargestellt. Das Maganzinierelement 20 besteht beispielsweise aus einem aus Medizinalblech gestanzten Schwert, auf welchem die Wundklammern übereinander gestapelt gehalten sind. Die beiden seitlichen Schneiden des Schwertes sind sägezahnartig gestaltet. Der Abstand zwischen zwei Positionierkerben 21 auf derselben Schneideseite entsprechen der Höhe der Wundklammern. Die schmälste Breite des Magazinierelementes 20, das heisst die Distanz zwischen zwei einander gegenüberliegenden Positionierkerben 21, entspricht dabei genau der Länge des zentrischen Einschnittes 13 in den Klemmbacken 10. Folglich sind sämtliche Wundklammern mit Ausnahme der untersten Wundklammer auf dem Magazinierelement 20 in der dargestellten Lage vollständig entspannt gehalten. Am untersten Ende ist das Magazinierelement so erweitert, dass die Wundklammer vollständig gespreizt gehalt ist. Das vordere Ende des Magazinierelementes 20 mündet in einer zweizinkigen Spreizgabel 22, die zwischen den beiden Zinken einen Freiraum 23 belassen. Bildet nun das Magazinierelement 20 Teil eines Wundklammernsetzgerätes, so kann man dieses über die beiden Wundränder hinweg auf die Haut aufsetzen, wobei die beiden hochgestülpten Wundränder im Freiraum 23 Platz haben. Werden nun die Wundklammern mittels nicht dargestellten Mitteln zurückgehalten, während das schwertförmige Magazinierelement um einen Schritt nach oben gezogen wird, so wird die unterste, gespreizte Wundklammer vom Magazinierelement abgeschoben und hält sogleich mit ihren beiden Andruckkanten 11 die beiden gegenüber liegenden Wundränder zusammen. Gleichzeitig werden alle darüber befindlichen Wundklammern um eine Positionierkerbendistanz nach unten bewegt. Auf diese Weise lassen sich die erfindungsgemässen Wundklammern lediglich durch die Auf- und Abbewegung des Magazinierelementes 20 einfach fördern, aufspreizen und auf die Wunde aufsetzen. Bedingung, dass dies jedoch funktioniert, ist die Stapelfähigkeit der Wundklammern und deren exakte Positionierung. Dies lässt sich mit der Erfindung auf äusserst einfache Art realisieren.

Das Prinzip der Magazinierung der Wundklammern, die als Mittel zur Halterung seitliche Ausnehmungen 13' aufweisen, erfolgt in fast identischer Weise. Bei der Ausführung gemäss der Figur 5 kann das Magazinierelement aus zwei parallelen schwertförmigen Teilen bestehen, die fast identisch jenen gemäss der Figur 7 sind. Zur exakten Führung können die beiden Magazinierelemente miteinander verbunden sein.

Dass die Andruckkanten 11 nicht einfach geradflächig sein müssen, demonstrieren die Wundklammern gemäss der Figur 7.

Diese sind wulstartig verdickt dargestellt. Obwohl in den Figuren die verschiedenen Ecken und Kanten scharfkantig gezeichnet sind, wird man vorzugsweise jene Kanten und Ecken, die mit der Baut des Patienten in Berührung kommen, teilweise gerundet gestalten.

## Patentansprüche

1. Einstückige, federelastische, bogenförmige Wundklammer (1) mit zwei zueinander gerichteten Andruckkanten (11), die von zwei über einen Steg (12) miteinander verbundenen Klemmbacken (10) gebildet sind und Mittel (13,13') zur stapelbaren Halterung auf einem sie durchsetzenden Magazinierelement aufweisen, wobei die Mittel (13, 13') mindestens ein Einschnitt (13) oder eine Ausnehmung (13') sind, dadurch gekennzeichnet, dass die mindestens eine Ausnehmung (13') beziehungsweise Einschnitt sowohl den Steg (12) als auch die Andruckkanten (11) der Klemmbacken (10) senkrecht zu der Verlaufsrichtung der Andruckkanten durchsetzen, und dass die Klemmbacken (10) mit Rückhalterippen (17) zur Halterung versehen sind, die parallel zur Richtung der Andruckkanten verlaufen.

2. Wundklammer nach Anspruch 1, dadurch gekennzeichnet, dass als Mittel zur stapelbaren Halterung ein länglicher, zentrischer, senkrecht zu den Andruckkanten (11) verlaufender Einschnitt (13) vorgesehen ist, der den Steg (12) vollständig und die beiden darunter befindlichen Klemmbacken (10) im Bereich der Andruckkanten (11) teilweise durchsetzt.

3. Wundklammer nach Anspruch 1, dadurch gekennzeichnet, dass als Mittel zur stapelbaren Halterung an mindestens einer der beiden die Wundklammer berandenden seitlichen Stirnflächen (14) der Wundklammer (1) eine Ausnehmung (13') eingelassen ist, die sowohl den Steg (12) als auch die beiden Klemmbacken (10) über die Andruckkanten (11) hinweg durchsetzt.

4. Wundklammer nach Anspruch 1, dadurch gekennzeichnet, dass die Mittel zur Halterung der Wundklammer (1) in einer positionierten Lage parallel zu und distanziert von den Andruckkanten (11) verlaufende Rückhalterippen (17) sind.

5. Wundklammer nach Anspruch 1, dadurch gekennzeichnet, dass die Andruckkanten (11) wellenförmig (18) gestaltet sind.

6. Wundklammer nach Anspruch 1, dadurch gekennzeichnet, dass auf dem Steg (12), und ausserhalb des Bereiches der Mittel zur stapelbaren Halterung, zwei vom Steg (12) abstehende Spreizelemente (19) angeordnet sind.

7. Wundklammer nach Anspruch 2, dadurch gekennzeichnet, dass die Länge des zentrischen Einschnittes (13) im Steg (12) grösser ist, als die Länge des Einschnittes (13) in den beiden Klemmbacken (10) zusammen.

8. Wundklammer nach Anspruch 3, dadurch gekennzeichnet, dass die Länge der Ausnehmung (13'), in mindestens einer der seitlichen Stirnflächen (14), im Steg (12) grösser ist als die Länge der Ausnehmung (13') in den beiden Klemmbacken (10) zusammen.

9. Wundklammer nach Anspruch 1, dadurch gekennzeichnet, dass diese aus einem transparenten Kunststoff gefertigt ist.

## Claims

1. A one-piece, spring-elastic arch-shaped wound clip (1) with two pressing edges (11) which are aligned to one another, which are formed by two clamping jaws (10) connected to one another via a web (12) and which comprise means (13, 13') for the stackable mounting on a magazining element interspersing these means, wherein the means (13, 13') are at least one incision (13) or a recess (13'), characterised in that the at least one recess (13') or incision intersperses the web (12) as well as the pressing edges (11) of the clamping jaws (10) perpendicular to the running direction of the pressing edges, and that the clamping jaws (10) are provided with retaining ribs (17) for the mounting, which run parallel to the direction of the pressing edges.

2. A wound clip according to claim 1, characterised in that as means for the stackable mounting, an elongate, centric incision (13) running perpendicularly to the pressing edges (11) is provided, this incision completely interspersing the web (12) and partly interspersing the two clamping jaws (10) located thereunder in the region of the pressing edges.

3. A wound clip according to claim 1, characterised in that as means for the stackable mounting, on at least one of the two lateral end faces (14) of the wound clip (1), which edges the wound clip, there is incorporated a recess (13') which intersperses the web (12) as well as the two clamping jaws (10) over the pressing edges (11).

4. A wound clip according to claim 1, characterised in that the means for mounting the wound clip (1) in a positioned location are retaining ribs (17) running parallel to and distanced from the pressing edges (11).

5. A wound clip according to claim 1, characterised in that the pressing edges (11) are formed wave-shaped.

6. A wound clip according to claim 1, characterised in that on the web (12) and outside the region of the means for the stackable mounting there are arranged two spreading elements (19) distance from the web (12).

7. A wound clip according to claim 2, characterised in that the length of the centric incision (13) in the web (12) is larger than the length of the incision (13) in the two clamping jaws (10).

8. A wound clip according to claim 3, characterised in that the length of the recess (13') in at least one of the lateral end faces (14), in the web (12) is larger than the length of the recess (13') in the two clamping jaws (10) together.

9. A wound clip according to claim 1, characterised in that this is manufactured from a transparent plastic.

## Revendications

1. Agrafe chirurgicale monocorps, à élasticité de ressort, de forme arquée (1) ayant deux arêtes de serrage (11) en vis-à-vis, qui sont formées de deux mâchoires de serrage (10) reliées entre elles par une entretoise (12) et présentent des moyens (13,13') pour la fixation avec empilement sur un élément de présentoir s'y insérant, tandis que les moyens (13,13') sont constitués d'au moins une entaille (13) ou un évidement (13'), caractérisée en ce que le ou les évidements (13') ou entailles, ainsi que l'entretoise (12) et les arêtes de serrage (11) des mâchoires de serrage (10) sont perpendiculaires à la direction des arêtes de serrage, et que les mâchoires de serrage (10) sont munies de nervures de retenue (17) destinées à les maintenir et qui ont une direction parallèle à celle des arêtes de serrage.

2. Agrafe chirurgicale selon la revendication 1, caractérisée en ce qu'est prévue comme moyen pour la fixation avec empilement une entaille (13) de forme allongée, centrée, perpendiculaire aux arêtes de serrage (11), qui traverse totalement l'entretoise (12) et partiellement les deux mâchoires de serrage (10) se trouvant en dessous dans la zone des arêtes de serrage (11).

3. Agrafe chirurgicale selon la revendication 1, caractérisée en ce que, comme moyen pour la fixation avec empilement, il est prévu sur au moins l'une des deux surfaces (14) définissant la bordure latérale de l'agrafe chirurgicale (1) un évidement (13') qui traverse aussi bien l'entretoise (12) que les deux mâchoires de serrage (10) au-dessus des arêtes de serrage (11).

4. Agrafe chirurgicale selon la revendication 1, caractérisée en ce que les moyens pour le maintien de l'agrafe chirurgicale (1) dans un positionnement défini sont des nervures de retenue (17) orientées parallèlement aux arêtes de serrage (11) et à une certaine distance de celles-ci.

5. Agrafe chirurgicale selon la revendication 1, caractérisée en ce que les arêtes de serrage (11) ont une forme ondulée (18).

6. Agrafe chirurgicale selon la revendication 1, caractérisée en ce que, sur l'entretoise (12) et à l'extérieur du domaine des moyens pour la fixation avec empilement, sont disposés deux éléments d'écartement (19) partant de l'entretoise (12).

7. Agrafe chirurgicale selon la revendication 2, caractérisée en ce que la longueur de l'entaille centrée (13) dans l'entretoise (12) est supérieure à la longueur de l'entaille (13) dans les deux mâchoires de serrage (10).

8. Agrafe chirurgicale selon la revendication 3, caractérisée en ce que la longueur de l'évidement (13'), dans au moins l'une des surfaces latérales (14), est supérieure dans l'entretoise (12) à la longueur de l'évidement (13') dans les deux mâchoires de serrage (10).

9. Agrafe chirurgicale selon la revendication 1, caractérisée en ce qu'elle est réalisée en une matière plastique transparente.
